# EUROPEAN PATENT APPLICATION

(11) **EP 3 088 377 A1**
(43) Date of publication of application: **02.11.2016**
(21) Application number: 15305675.9
(22) Date of filing: 30.04.2015
(51) Int. Cl.: C07C 51/235

(54) **PROCESS FOR THE PREPARATION OF AN ALDARIC ACID OR A SALT THEREOF**

(71) Applicant: Rhodia Operations, 75009 Paris (FR); Université Claude Bernard Lyon 1, 69100 Villeurbanne (FR); CENTRE NATIONAL DE LA RECHERCHE SCIENTIFIQUE, 75794 Paris Cedex 16 (FR)
(72) Inventor: MARION, Philippe, 69390 Vernaison (FR); DERRIEN Elie,, 69003 Lyon, (FR); PINEL Catherine,, 69007 Lyon, (FR); BESSON Michèle,, 01700 Les Echets, (FR)
(74) Representative: Vande Gucht, Anne

(57) **Abstract**

The invention relates to a process for the preparation of an aldaric acid or a salt thereof.

## Description

### Technical Field

The invention relates to a process for the preparation of an aldaric acid or a salt thereof comprising reacting a cellulosic material hydrolysate comprising a reducing sugar with oxygen in the presence of an oxidation catalyst.

### Prior Art

Sugars are primary constituents of heterogeneous complex cellulosic biomass. They can be produced and recovered after selective hydrolysis of the biomass, sulfuric acid being known as a convenient reagent for this transformation. Intense research has been done on the highly efficient and selective conversion of glucose into high value-added chemicals. Gluconic and glucaric acids and their salts can be obtained by oxidation of aqueous glucose solutions using atmospheric oxygen or air in the presence of catalysts. Most of the investigations were oriented towards the selective oxidation of glucose to gluconic acid and gluconate, which is an important intermediate in food industry and pharmaceutical applications, and as a water-soluble cleaner for removing calcareous deposits (Besson, M. et al., Chem. Rev. 114, 1827 (2014)).

An increased interest was given to glucaric acid. This compound has been specified among the twelve most promising sugar-based building blocks for the chemical industry by the US Department of Energy. It is a promising raw material for biodegradable detergents, metal complexation agents and monomers. However, there are very few studies on the oxidation of glucose to glucaric acid, and the current synthesis methods are far from satisfactory.

US 2,427,168 describes the selective oxidation of glucose to glucarate in aqueous alkaline medium over 5 % Pt/C catalyst. US 2011/0306790 describes the selective oxidation of glucose to glucaric acid in aqueous solution over Au-Pt supported catalysts. US 2,472,168 and US 2011/0306790 employ pure D glucose as starting material.

However, in the prior art only oxidation of pure glucose as starting material to glucaric acid is described. Therefore, there is a need for a process enabling the direct utilization of cellulosic material to produce an aldaric acid or a salt thereof.

### Summary of the Invention

The authors of the present invention surprisingly found that an aldaric acid or a salt thereof can be prepared by a process which comprises reacting a cellulosic material hydrolysate comprising a reducing sugar with oxygen in the presence of an oxidation catalyst.

Therefore, the invention relates to a process for the preparation of an aldaric acid or a salt thereof, which process comprises reacting a cellulosic material hydrolysate comprising a reducing sugar with oxygen in the presence of an oxidation catalyst.

The invention also relates to the use of an oxidation catalyst in the process for the preparation of an aldaric acid or a salt thereof.

### Definitions

For purposes of the present description, certain terms are intended to have the following meanings.

The term "comprising" includes "consisting essentially of" and also "consisting of".

In addition, if the term "about" is used before a quantitative value, the present teachings also include the specific quantitative value itself, unless specifically stated otherwise. As used herein, the term "about" preferably refers to a ±10 % variation from the nominal value unless specifically stated otherwise.

### Brief Description of the Figures

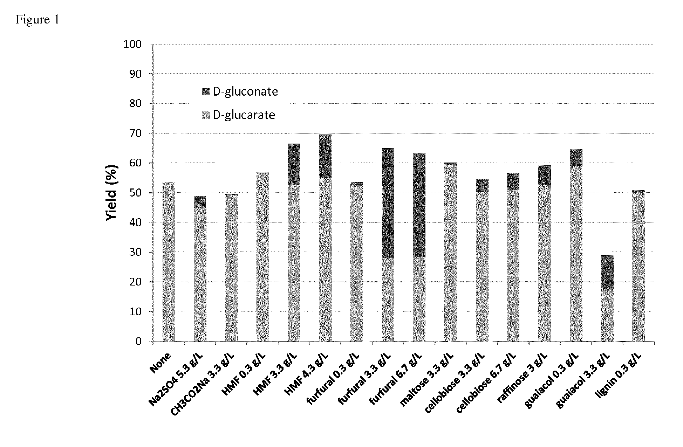

Figure 1 shows the results of examples 2 and 3. The yields reported after 24 hours reaction are calculated on the basis of initial molar glucose concentration.

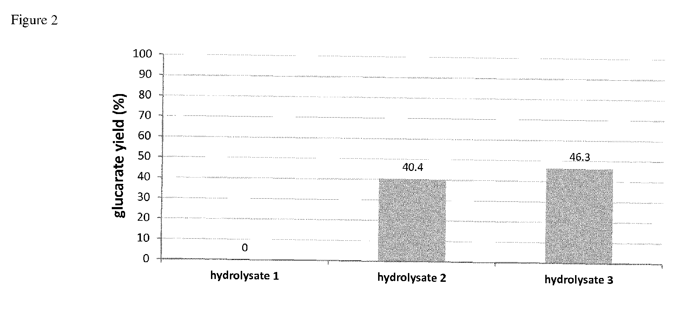

Figure 2 shows the results of the aerobic oxidation of D-glucose in hydrolysates over Pt/C (example 4). The yields are calculated on the basis of initial molar glucose concentration.

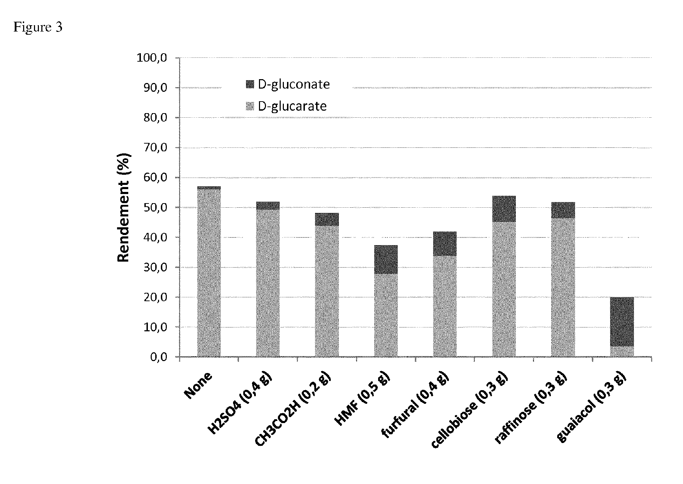

Figure 3 shows the results of examples 6 and 7. The yields reported after 8 hours reaction are calculated on the basis of initial molar glucose concentration.

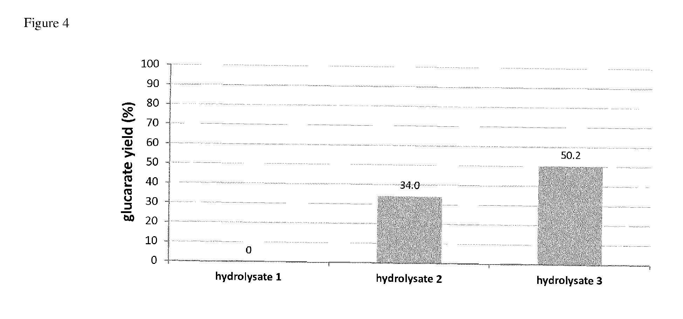

In Figure 4, the results of the Au-Pt catalyzed aerobic oxidation of glucose in hydrolysates (example 8) are shown. The yields are calculated on the basis of initial molar glucose concentration.

### Detailed Description of the Invention

The inventors of the present invention have surprisingly observed that an aldaric acid or a salt thereof can be produced by reacting a cellulosic material hydrolysate comprising a reducing sugar with oxygen in the presence of an oxidation catalyst.

Therefore, the present invention relates to a process for the preparation of an aldaric acid or a salt thereof, which process comprises reacting a cellulosic material hydrolysate comprising a reducing sugar with oxygen in the presence of an oxidation catalyst.

The cellulosic material hydrolysate used in the invented process is a material comprising a reducing sugar obtainable by hydrolysis of cellulosic material. Accordingly, it usually contains one or more characteristic impurities (which can be viewed as "markers"), namely unreacted cellulosic material and/or one or more impurities that can be generated by hydrolysis reaction of cellulosic material. In practice, the cellulosic material hydrolysate is advantageously obtained by hydrolysis of cellulosic material.

Cellulosic material as used herein includes renewable resources such as energy crops, plant biomass, agricultural wastes, forestry residues, sugar processing residues and plant-derived household wastes. More generally, renewable resources that may be used in accordance with the present invention include any renewable organic method that includes a source of hemicellulose such as, for example, switch grass, straw, oat halves, miscanthus, cassava, trees, vegetation, and crop residues. Other sources can include, for example, waste materials (e.g. spent paper, green waste, municipal waste, etc.).

The hydrolysis of cellulosic material is not limited to a specific method. A convenient reagent for the hydrolysis is sulfuric acid (R.J. Chimentao et al., Carbohydr. Polym. 2014, 111, 116-124).

In the context of the present invention a reducing sugar is any sugar that either has an aldehyde group or is capable of forming one in solution through isomerism. Examples of reducing sugars include monosaccharides, disaccharides, oligosaccharides and polysaccharides, as long as they have an open chain form with an aldehyde group or a free hemiacetal group. Monosaccharides which contain an aldehyde group are known as aldoses, and those with a ketone group are known as ketoses. Aldoses are grouped according to the number of carbon atoms and the carbon chain as dioses, trioses, tetroses, pentoses and hexoses. Glycoaldehyde is a diose and glyceraldehyde is a triose. Examples for tetroses include erythrose and triose, examples of pentoses include ribose, arabinose, xylose and lyxose and examples of hexoses include allose, altrose, glucose, mannose, gulose, idose, galactose and talose.

Also many disaccharides, like lactose and maltose have a reducing form as one of the two units may have an open chain form with an aldehyde group.

The cellulosic material hydrolysate comprises a reducing sugar. For the avoidance of doubt, "a reducing sugar" as herein used denotes one or more reducing sugars. Advantageously, the cellulosic material hydrolysate contains several reducing sugars; in particular it contains a plurality of reducing sugars obtained by hydrolysis of cellulosic material.

In one embodiment of the present invention the reducing sugar is selected from aldopentoses and aldohexoses. Preferably, the aldopentoses according to the present invention include arabinose, xylose, ribose and xylose. Most preferred are arabinose and xylose.

Preferably, the aldohexoses according to the present invention comprise allose, altrose, glucose, mannose, gulose, idose, galactose and talose. Most preferred are glucose, mannose and galactose.

In one embodiment of the present invention, the reducing sugar is or comprises glucose.

As used herein, the reducing sugar refers to all possible stereoisomers of said sugar. Therefore, the "D-" and "L-" prefixes that are used for describing the stereoisomerism of monosaccharides are both encompassed if the term reducing sugar is used.

Furthermore, the present inventors found that the reaction of the cellulosic material hydrolysate comprising a reducing sugar with oxygen can be adversely affected. In particular, the time required for the conversion of a reducing sugar to an aldaric acid can be significantly increased. Furthermore, the glucarate yield can be significantly decreased. Upon further investigations, the inventors found that reducing sugar obtained by hydrolysis of cellulosic material generally contains impurities besides the desired reducing sugar. More precisely, the inventors analyzed various lots of reducing sugar obtained by hydrolysis of cellulosic material and found that these hydrolysates generally contained sulfate(s), acetate(s), 5-hydroxymethylfurfural (HMF), furfural, maltose, cellobiose, raffinose and lignin besides the desired reducing sugar.

Therefore, in one embodiment of the present invention, the amount of one or more hydrolysate-type impurities is reduced prior to the reaction with oxygen. In order to reduce the amount of hydrolysate-type impurities, the hydrolysate may be treated prior to the reaction with oxygen by at least one treatment step selected from ion exchange chromatography, active carbon treatment, and distillation (concentration). In a preferred embodiment, the hydrolysate is treated by ion exchange chromatography following active carbon treatment which may be followed by distillation. Other treatments for reducing the amount of hydrolysate-type impurities in a hydrolysate are known to the skilled person.

Upon further investigation, the inventors found that surprisingly only specific impurities adversely affect the reaction of the cellulosic material hydrolysate comprising a reducing sugar with oxygen, namely HMF and furfural. The other impurities were found to have significantly less or even no influence on the reaction with oxygen.

In one embodiment of the present invention, the cellulosic material hydrolysate is in the form of a solution. In a preferred embodiment, said solution is an aqueous solution.

The term "solution" as used herein also includes a dispersion, as long as the cellulosic material hydrolysate comprising a reducing sugar can be reacted with oxygen in the presence of an oxidation catalyst.

Upon further investigations, the inventors found that nevertheless a certain residual amount of HMF and furfural is acceptable during the subsequent reaction with oxygen. This finding is particularly relevant on an industrial scale because removal of undesired impurities after the hydrolysis step is time consuming and costly. It is of advantage if the impurities can remain in the solution up to a concentration where they have no or only a little adverse influence on the reaction with oxygen. Therefore, in an embodiment, the amount of HMF and the amount of furfural are left unchanged prior to the reaction of the cellulosic material hydrolysate with oxygen. In a subembodiment, the amount of sulfate(s), the amount of acetate(s), the amount of HMF, the amount of furfural, the amount of maltose, the amount of cellobiose, the amount of raffinose and the amount of lignin are all left unchanged prior to the reaction of the cellulosic material hydrolysate with oxygen; still more particularly, the cellulosic material hydrolysate is not submitted to any purification step before being reacted with oxygen.

As above indicated, the cellulosic material hydrolysate used in the invented process contains generally one or more sulfates. The weight amount of sulfate (understand the total weight amount of the sulfates, if several) expressed as sulfate anion, based on the weight amount of reducing sugar (understand the total weight amount of reducing sugars, if several), is very often above 0.001; it is often above 0.01 or even above 0.05, and may sometimes exceed 0.15 or even 0.30. On the other hand, it is usually below 1.00, very often below 0.50 and often below 0.20. Typical sulfate : reducing sugar weight ratios range from 0.05 to 0.15.

The cellulosic material hydrolysate used in the invented process also contains generally one or more acetates. The weight amount of acetate (understand the total weight amount of the acetates, if several) expressed as acetate anion, based on the weight amount of reducing sugar, is very often above 0.001; it is often above 0.01 or even above 0.03, and may sometimes exceed 0.10 or even 0.30. On the other hand, it is usually below 1.00, very often below 0.50 and often below 0.20. Typical acetate : reducing sugar weight ratios range from 0.03 to 0.10.

The cellulosic material hydrolysate used in the invented process also contains generally maltose. The weight amount of maltose, based on the weight amount of reducing sugar, is very often above 0.001; it is often above 0.01 or even above 0.03, and may sometimes exceed 0.10 or even 0.30. On the other hand, it is usually below 1.00, very often below 0.50 and often below 0.20. Typical maltose : reducing sugar weight ratios range from 0.03 to 0.10.

The cellulosic material hydrolysate used in the invented process also contains generally cellobiose. The weight amount of cellobiose, based on the weight amount of reducing sugar, is very often above 0.001; it is often above 0.01, and may sometimes exceed 0.15 or even 0.30. On the other hand, it is usually below 1.00, very often below 0.50 and often below 0.20. Typical cellobiose : reducing sugars weight ratios range from 0.03 to 0.15.

The cellulosic material hydrolysate used in the invented process also contains generally raffinose. The weight amount of raffinose, based on the weight amount of reducing sugar, is very often above 0.001; it is often above 0.01, and may sometimes exceed 0.15 or even 0.30. On the other hand, it is usually below 1.00, very often below 0.50 and often below 0.20. Typical raffinose : reducing sugar weight ratios range from 0.03 to 0.15.

The cellulosic material hydrolysate used in the invented process also contains generally lignin. The weight amount of lignin, based on the weight amount of reducing sugar, is very often above 0.0001; it is often above 0.0005, and may sometimes exceed 0.0030 or even 0.0150. On the other hand, it is very often below 0.0500 and often below 0.0200. Typical lignin : reducing sugar weight ratios range from 0.0020 to 0.0100.

The cellulosic material hydrolysate used in the invented process also contains generally 5-hydroxymethylfurfural (HMF). The weight amount of HMF, based on the weight amount of reducing sugar, is very often above 0.0001 or even above 0.001; it is often above 0.01 or even above 0.03 and may sometimes exceed 0.05 or even 0.10. On the other hand, it is very often below 0.60 and often below 0.25. Typical HMF : reducing sugar weight ratios range from 0.03 to 0.10. In some embodiments, the solution of the cellulosic material hydrolysate comprising a reducing sugar used in the reaction with oxygen contains less than about 1.0 g/l of HMF, possibly less than about 0.9 g/l, less than about 0.8 g/l, less than about 0.7 g/l or less than about 0.6 g/l of 5-hydroxymethylfurfural.

The cellulosic material hydrolysate used in the invented process also contains generally furfural. The weight amount of furfural, based on the weight amount of reducing sugar, is very often above 0.0001 or even above 0.001; it is often above 0.01 or even above 0.03 and may sometimes exceed 0.05 or even 0.10. On the other hand, it is very often below 0.60 and often below 0.25. Typical furfural: reducing sugar weight ratios range from 0.03 to 0.10.

In some embodiments, the solution contains the above lower concentrations of HMF and furfural at the same time, whereby all upper limits of the concentration of HMF may be combined with all upper limits of the concentration of furfural. The solution may contain less than about 1.0 g/l of 5-hydroxymethylfurfural and less than about 1.0 g/l of furfural; it may contain less than about 0.9 g/l of 5-hydroxymethylfurfural and less than about 0.9 g/l of furfural; it may contain less than about 0.6 g/l of 5-hydroxymethylfurfural and less than about 0.6 g/l of furfural.

In order to reduce the amount of 5-hydroxymethylfurfural and/or furfural, the solution may be treated prior to the reaction with oxygen by at least one treatment step selected from ion exchange chromatography, active carbon treatment, and distillation (concentration). In a preferred embodiment, the solution is treated by ion exchange chromatography following active carbon treatment which may be followed by distillation. Other treatments for reducing the amount of HMF and/or furfural in a solution are known to the skilled person.

The oxygen source in the process according to the present invention is not particularly limited. Preferably, the oxygen is supplied as air, oxygen enriched air, or oxygen, with one or more other constituents substantially inert to the reaction. Most preferably, the oxygen is supplied as air. The oxygen or oxygen-containing gas can be bubbled through the solution, for example under ambient pressure or at increased pressure.

In some embodiments, the air flow is in the range of about 5 to about 5,000 ml per minute, preferably about 100 to about 4,000 ml per minute, more preferably about 150 to about 2,000 ml per minute and most preferably in the range of about 200 to about 1,500 ml per minute, each in respect to about 100 to about 1000 ml of solution of reducing sugars.

The oxidation catalyst according to the present invention is not particularly limited.

In some embodiments, the oxidation catalyst comprises at least one noble metal. The case being, the mole amount of reducing sugar (understand the total mole amount of reducing sugars, if several), based on the total mole amount of noble metal (understand the total mole amount of noble metals, if several) contained in the oxidation catalyst, is advantageously below 1000; it is preferably below 500, and much preferably below 200. On the other hand, it is advantageously above 10, preferably above 20 and much preferably above 50. Typical reducing sugar : noble metal mole ratios range from 50 to 200.

In a preferred embodiment the noble metal is selected from the group consisting of Au, Pt and Pd.

In one embodiment, the oxidation catalyst is a supported catalyst. Preferably, the catalyst support is selected from the group consisting of carbon, ceria, titania and zirconia.

In another embodiment, the oxidation catalysts are Pt supported on active carbon, TiO₂, ZrO₂ or CeO₂ and other metal oxides.

In a preferred embodiment, the oxidation catalyst is Pt/C.

In another preferred embodiment, the oxidation catalyst comprises Au and Pt.

In an even more preferred embodiment, the oxidation catalyst is Au-Pt/ZrO₂.

The catalyst preparation methods include metallic sol immobilization, wet impregnation, incipient wetness impregnation and deposition-precipitation.

Deposition of Au and Pt can be successive or simultaneous.

The Pt:Au ratio may vary, for example, from about 100:1 to about 1:10, from about 50:1 to about 1:10, from about 10:1 to about 1:10 or more preferably from about 3:1 to about 1:4. More preferably still, the Pt:Au molar ratio may vary for example from about 3:1 to about 1:2. Even more preferably, the molar ratio of Pt:Au is in the range of about 2:1 to about 1:2, for example about 1:1.

The total metal loading on the final catalyst (i.e., excluding any metal that is from the support) is generally less than about 10 wt.-% relative to the total catalyst weight. Preferably, the total metal loading ranges from about 1 to about 8 wt.-% relative to the total catalyst weight.

In one embodiment of the present invention the oxidation catalyst is Pt/C and during the reaction with oxygen the solution is maintained at a basic pH. In a preferred embodiment, the pH of this solution during the reaction with oxygen is maintained at equal to or above about 8, more preferably in the range of about 8 to about 10, and most preferably at about 9.

In another embodiment, the oxidation catalyst is Au-Pt/ZrO₂ and during the reaction with oxygen the pH of the solution is not regulated and decreases to a value of about 2 as the reaction proceeds.

In one embodiment, the oxidation catalyst is about 3.5 wt.-% Au and about 3.5 wt.-% Pt/ZrO₂.

Generally, the temperature during the reaction with oxygen is at least about 10°C, such as in the range of about 15°C to about 150°C, preferably in the range of about 25°C to about 125°C, more preferably in the range of about 25°C to about 100°C.

If the reaction with oxygen is conducted in a solution, the temperature of the solution during the reaction with oxygen is at least about 10°C, preferably in the range of about 25°C to the boiling point of the solution. The temperature of the solution in the reaction with oxygen can for example be from about 50°C to the boiling point of the solution, preferably in the range of about 25°C to about 125°C, or about 25°C to about 100°C.

Typically, oxygen-containing gas pressure is in the range of about 1 to about 150 bar, preferably in the range of about 1 to about 80 bar, and most preferably about 40 bar.

In general, the oxidation reaction can be conducted in a batch, or semibatch reactor, or any other design that allows heterogeneous catalytic reactions. It should be understood that a reducing sugar, oxygen, any solvent, and the oxidation catalyst may be introduced into a suitable reactor separately or in various combinations. Particularly preferred is a pressurized Hastelloy reactor.

The initial concentration of reducing sugar can be in the range of about 1 to about 5,000 mmol/l, preferably about 10 to about 2,000 mmol/l, even more preferably in the range of about 50 to about 1,000 mmol/l and most preferably in the range of about 50 to about 500 mmol/l, such as about 250 mmol/l of the cellulosic material hydrolysate.

If the reaction is conducted in a solution, the initial concentration of reducing sugar can be in the range of about 1 to about 5,000 mmol/l, preferably about 10 to about 2,000 mmol/l, even more preferably in the range of about 50 to about 1,000 mmol/l and most preferably in the range of about 50 to about 500 mmol/l, such as about 250 mmol/l of the solution.

In a preferred embodiment, the parameters are set to an initial reducing sugar concentration of about 250 mmol/l of the solution, a temperature of about 100°C, and an oxygen-containing gas, preferably air pressure of about 40 bar.

In another embodiment, the parameters are set to an initial reducing sugar concentration of about 250 mmol/l of the solution, a temperature of about 60°C, a pH of about 9 and an oxygen-containing gas, preferably air flow of about 500 ml per minute.

In one embodiment, during the reaction with oxygen, the solution is stirred at about 100 to about 2,000 rpm, preferably about 120 to about 1,200 rpm.

In one embodiment of the present invention, the cellulosic material hydrolysate comprises at least two reducing sugars that have a different number of carbon atoms.

Preferably, the at least two reducing sugars comprises aldopentoses and aldohexoses. Preferably, the aldopentoses according to the present invention include arabinose, lyxose, ribose and xylose. Most preferred are arabinose and xylose.

Preferably, the aldohexoses according to the present invention comprise allose, altrose, glucose, mannose, gullose, idose, galactose and tallose. Most preferred are glucose, mannose and galactose.

Another aspect of the present invention is the use of an oxidation catalyst in the above described process.

The following examples are intended only to exemplify the invention and are not intended to limit the scope of the claimed invention.

### Examples

### Analysis

Samples of reaction groups were analyzed using a Metrohm 850 ion chromatograph. For examples 2, 3, 6 and 7, the products were separated on a IonPac AS11-HC column and then quantified by conductrometric detection. For examples 4 and 8, the products were separated on a CarboPac^{®} PA1 column and then quantified by amperometric detection. External calibration with standards was used to determine glucose conversion and yields of aldarates.

### Example 1 - Preparation of 4.5 wt.-% Pt/C catalyst

In a 500 ml round bottomed flask, 19.91 g of dry carbon (CECA L3S) is dispersed in 200 ml ultrapure water under nitrogen atmosphere. An aqueous solution of H₂PtCl₆ (H₂PtCl₆•6H₂O 99.99 % Sigma Aldrich) containing 1.30 g Pt is slowly added to the suspension under stirring which is maintained for 4 hours. This suspension is cooled to 0°C. 55 ml of 37 wt.-% formaldehyde and 30 ml of 30 wt.-% KOH are subsequently added dropwise. After 16 hours of stabilization, the solution is filtrated, washed with deionized water and dried under nitrogen flow at 60°C to obtain 4.5 wt.-% Pt/C.

### Example 2 - Oxidation of pure D-glucose

300 ml of a 0.25 M D-glucose solution (D-glucose monohydrate 99 % Alfa Aesar) in deionized water are loaded in a 1.0 l double jacket glass reactor equipped with mechanical agitation, gas bubbling and controlled alkaline pH assured by automatic injection of 10 wt.-% NaOH. Nitrogen is bubbled through the solution during 15 minutes, then 2.07 g of the catalyst according to example 1 is added and the reactor is heated to 60°C. The pH regulation is then set to 9 and the reaction is started by switching from nitrogen to 500 ml per minute air and setting agitation to 1,200 rpm. Samples are filtrated through 0.45 µm PVDF membranes, diluted in ultrapure water and analyzed by ion chromatography.

After 24 hours, glucose and gluconate are totally converted, which results are shown in Table 1.

### Example 3 - Oxidation of D-glucose in a solution containing hydrolysate -type impurities.

The procedure of example 2 is repeated with addition of a definite amount of hydrolysate-type impurities to the initial reaction mixture. Guaiacol is a soluble lignin model impurity.

The results of the experiments are shown in Table 1.

### Example 4 - Oxidation of D-glucose contained in pinewood hydrolysate

The procedure of the example 2 is applied to a pinewood hemicellulose hydrolysate and the results of the experiments are shown in Table 2. Hydrolysate 1 was treated by filtration and concentration. Hydrolysate 2 was treated by filtration, ion exchange chromatography and concentration. Hydrolysate 3 was treated by filtration, ion exchange chromatography, active carbon treatment and concentration.

### Example 5 - Preparation of 3.5 wt.-% Au - 3.5 wt.-% Pt/ZrO₂ catalyst

In a 1.0 l glass reactor equipped with mechanical agitation, 7.53 g of dry ZrO₂ (Mel Chemicals ZXO) is dispersed in 500 ml ultrapure water under nitrogen atmosphere. An aqueous solution of H₂PtCl₆ (H₂PtCl₆ • 6H₂O 99.99 % Sigma Aldrich) and HAuCl₄ (HAuCl₄ • 3H₂O 99.9+% Sigma Aldrich) containing 0.284 Pt and 0.284 g Au is added to the suspension under stirring which is maintained for 1 hour (400 rpm). The suspension is then cooled to 0°C and a 100 ml aqueous solution containing 1.25 g Nab4 (NaBH₄ > 98 % Sigma Aldrich) is added dropwise under vigorous agitation (800 rpm). After 3 hours of stabilization, the solution is filtrated, washed with deionized water and dried under nitrogen flow at 60°C to obtain 3.5 wt.-% Au - 3.5 wt.-% Pt/ZrO₂.

### Example 6 - Oxidation of pure D-glucose

100 ml of a 0.25 M D-glucose solution (D-glucose monohydrate 99 % Alfa Aesar) in deionized water are loaded in a 300 ml Parr Hastelloy reactor equipped with mechanical agitation, sampling tube, pressure and temperature regulation. Argon is bubbled through the solution during 15 minutes, then 0.883 g of the catalyst according to example 5 is added and the reactor is sealed. The reactor is flushed three times with 5 bar argon, then the temperature is raised to 100°C. The reaction is started by pressurizing the reactor to 40 bar air and setting agitation to 1,200 rpm. Samples are filtrated through 0.45 µm PVDF membranes, diluted in ultrapure water and analyzed by ion chromatography. After 8 hours, glucose is totally converted, which results of the experiment are shown in Table 3.

### Example 7 - Oxidation of D-glucose in a solution containing hydrolysate -type impurities.

The procedure of the example 6 is repeated with assition of a definite amount of hydrolysate-type impurities to the initial reaction mixture. The results of the experiments are shown in Table 3.

### Example 8 - Oxidation of D-glucose contained in pinewood hydrolysates

The procedure of example 6 is applied to a pinewood hemicellulose hydrolysate and the results of the experiments are shown in Table 4. Hydrolysate 1 was treated by filtration and concentration. Hydrolysate 2 was treated by filtration, ion exchange chromatography and concentration. Hydrolysate 3 was treated by filtration, ion exchange chromatography, active carbon treatment and concentration.

**Table 1**

| Results of the Pt-catalyzed aerobic oxidation of pure D-glucose in the presence of hydrolysate-type or lignin model impurities. | | | | |
|---|---|---|---|---|
| Yields reported after 24 h reaction are calculated on the basis of initial molar glucose concentration. | | | | |
| Example | Impurity (Concentration) | Mass (g) | D-gluconate yield (%) | D-glucarate yield (%) |
| 2 | None | | 0.0 | 53.6 |
| 3 | Na₂SO₄ 5.3 g/L | 1.6 | 4.1 | 44.9 |
| | CH3CO2Na 3.3 g/L | 1.0 | 0.3 | 49.3 |
| | HMF 0.3 g/L | 0.1 | 0.5 | 56.4 |
| | HMF 3.3 g/L | 1.0 | 14.1 | 52.4 |
| | HMF 4.3 g/L | 1.3 | 14.6 | 55.0 |
| | furfural 0.3 g/L | 0.1 | 1.0 | 52.5 |
| | furfural 3.3 g/L | 1.0 | 36.9 | 28.0 |
| | furfural 6.7 g/L | 2.0 | 35.1 | 28.3 |
| | maltose 3.3 g/L | 1.0 | 1.0 | 59.2 |
| | cellobiose 3.3 g/L | 1.0 | 4.5 | 50.1 |
| | cellobiose 6.7 g/L | 2.0 | 5.7 | 50.9 |
| | raffinose 3 g/L | 0.9 | 6.7 | 52.5 |
| | guaiacol 0.3 g/L | 0.1 | 5.8 | 58.9 |
| | guaiacol 3.3 g/L | 1.0 | 11.8 | 17.2 |
| | lignin 0.3 g/L | 0.1 | 0.7 | 50.2 |

**Table 2**

| Results of the Pt-catalyzed aerobic oxidation of glucose in pinewood hydrolysates | | | | | | | | |
|---|---|---|---|---|---|---|---|---|
| Yields are calculated on the basis of initial molar glucose concentration. | | | | | | | | |
| Example | Starting solution | Color | [glucose]₀ (mM) | [HMF] (g/L) | [furfural] (g/L) | Conductivity (mS/cm) | Reaction time (h) | glucarate yield (%) |
| 4 | hydrolysate 1 | yellow | 43.5 | 1.1 | 0 | 12 | 48 | 0 |
| | hydrolysate 2 | colorless | 25.8 | 0.5 | 0 | 0.00014 | 34 | 40.4 |
| | hydrolysate 3 | colorless | 27.7 | 0.0028 | 0 | 0.00034 | 34 | 46.3 |

**Table 3**

| Results of the Au-Pt-catalyzed aerobic oxidation of D-glucose in the presence of hydrolysate-type or lignin model impurities. | | | | |
|---|---|---|---|---|
| Yields reported after 8 h reaction are calculated on the basis of initial molar glucose concentration. | | | | |
| Example | Impurity (Concentration) | Mass (g) | D-gluconic acid yield (%) | D-glucaric acid yield (%) |
| 6 | None | | 1.0 | 56.0 |
| 7 | H₂SO₄ (3.9 g/L) | 0.4 | 2.7 | 49.2 |
| | CH₃CO₂H (2.3 g/L) | 0.2 | 4.3 | 43.8 |
| | HMF (4.8 g/L) | 0.5 | 9.5 | 27.8 |
| | furfural (3.6 g/L) | 0.4 | 8.2 | 33.8 |
| | cellobiose (3.3 g/L) | 0.3 | 8.8 | 45.1 |
| | raffinose (3.3 g/L) | 0.3 | 5.3 | 46.5 |
| | guaiacol (3.3 g/L) | 0.3 | 16.3 | 3.6 |

**Table 4**

| Results of the Au-Pt-catalyzed aerobic oxidation of glucose in pinewood hydrolysates. | | | | | | | | |
|---|---|---|---|---|---|---|---|---|
| Yields are calculated on the basis of initial molar glucose concentration. | | | | | | | | |
| Example | Starting solution | Color | [glucose]₀ (mM) | [HMF] (g/L) | [furfural] (g/L) | Conductivity (mS/cm) | Reaction time (h) | glucarate yield (%) |
| 8 | hydrolysate 1 | yellow | 43.5 | 1.1 | 0 | 12 | 48 | 0 |
| | hydrolysate 2 | colorless | 25.8 | 0.5 | 0 | 0.00014 | 34 | 34.0 |
| | hydrolysate 3 | colorless | 27.7 | 0.0028 | 0 | 0.00034 | 10 | 50.2 |

## Claims

1. A process for the preparation of an aldaric acid or a salt thereof, wherein the process comprises reacting a cellulosic material hydrolysate comprising a reducing sugar with oxygen in the presence of an oxidation catalyst.

2. The process according to claim 1, wherein the amount of one or more hydrolysate-type impurities is reduced prior to the reaction with oxygen.

3. The process according to claim 2, wherein the reduction of hydrolysate-type impurities is conducted by at least one of the following treatment steps: (1) ion exchange chromatography, (2) active carbon treatment, and (3) distillation.

4. The process according to any one of claims 2 and 3, wherein the hydrolysate-type impurities comprise furfural and/or 5-hydroxymethylfurfural.

5. The process according to any one of the preceding claims, wherein the cellulosic material hydrolysate is in the form of a solution, preferably an aqueous solution.

6. The process according to claim 5, wherein the solution prior to the reaction with oxygen contains less than about 1.0 g/L of 5-hydroxymethylfurfural, preferably less than about 0.9 g/L of 5-hydroxymethylfurfural and most preferably less than about 0.6 g/L of 5-hydroxymethylfurfural.

7. The process according to any one of claims 5 and 6, wherein the solution prior to the reaction with oxygen contains less than about 1.0 g/L of furfural.

8. The process according to any one of the preceding claims, wherein the oxygen is supplied as air, oxygen-enriched air, or oxygen, with one or more other constituents substantially inert to the reaction.

9. The process according to any one of the preceding claims, wherein the oxidation catalyst comprises at least one noble metal, preferably selected from the group consisting of Au, Pt and Pd.

10. The process according to any one of the preceding claims, wherein the oxidation catalyst is a supported catalyst and the catalyst support is preferably selected from the group consisting of carbon, ceria, titania and zirconia.

11. The process according to any one of the preceding claims, wherein the oxidation catalyst is selected from the group consisting of Pt/C and Au-Pt/ZrO₂.

12. The process according to any one of claims 5 to 11, wherein the oxidation catalyst is Pt/C and during the reaction with oxygen the solution is maintained at a basic pH, preferably at a pH of equal to or above about 8.

13. The process according to any one of claims 5 to 11, wherein the oxidation catalyst is Au-Pt/ZrO₂ and during the reaction with oxygen the pH of the solution is not regulated.

14. The process according to any one of the preceding claims, wherein the cellulosic material hydrolysate comprises at least two reducing sugars that have a different number of carbon atoms, preferably selected from aldopentoses and aldohexoses.

15. Use of an oxidation catalyst in the process according to any one of claims 1 to 14.
